# EUROPÄISCHE PATENTSCHRIFT

(11) **EP 0 781 745 B1**
(45) Veröffentlichungstag und Bekanntmachung des Hinweises auf die Patenterteilung: **03.05.2000**
(21) Anmeldenummer: 96710019.9
(22) Anmeldetag: 21.12.1996
(51) Int. Cl.: C07C 17/08, C07C 17/20, C07C 19/08, C07B 39/00, B01J 23/14

(54) **Katalytische Gemische auf Basis von teilfluorierten Titan- und Zinnhalogeniden und ihre Verwendung zur Herstellung fluorierter organischer Verbindungen**
Catalytic mixture based on partially fluorinated titane and zinc halogenides and their application in the preparation of fluorinated organic compounds
Mélange catalytique à base d'halogénures de titane et de zinc partiellement fluorés ainsi que leur application à la préparation de produits organiques fluorés

(30) Priorität: 28.12.1995 DE 19548999
(43) Veröffentlichungstag der Anmeldung: 02.07.1997
(73) Patentinhaber: Solvay Fluor und Derivate GmbH, D-30173 Hannover (DE)
(72) Erfinder: Braun, Max, Dr., 30900 Wedemark (DE); Rudolph, Werner, Dr., 30559 Hannover (DE); Palsherm, Stefan, 30890 Barsinghausen (DE)
(74) Vertreter: Lauer, Dieter, Dr.

(56) Entgegenhaltungen:
- EP-A- 0 574 077
- EP-A- 0 675 096

## Beschreibung

Die vorliegende Erfindung betrifft katalytische Gemische auf Basis von mindestens teilfluoriertem Titantetrachlorid und Zinntetrachlorid und ihre Verwendung zur Herstellung fluorierter organischer Verbindungen.

Es ist bereits eine Vielzahl von Katalysatoren bekannt, die für die Anlagerung von Fluorwasserstoff (HF) und für den Chlor-Fluor-Austausch mittels Fluorwasserstoff geeignet sind. Die europäische Patentanmeldung EP-0 522 638 A1 offenbart beispielsweise ein Verfahren zur Herstellung von 1,1-Dichloro-1,3,3,3-Tetrafluoropropan aus der entsprechenden Hexachloropropan-Verbindung. Als Katalysatoren, die die Reaktion fördern, werden Metalle der Gruppen IIIa, IVa und b, Va und b sowie der Gruppe VIb des Periodensystems der Elemente und ihre Mischungen zitiert. Besonders gut geeignet sind Titanderivate, Tantalderivate, Molybdänderivate, Borderivate, Zinnderivate und Antimonderivate. Zinnderivate und Antimonderivate sind als besonders bevorzugt hervorgehoben.

Die europäische Patentanmeldung EP-0 415 814 A1 offenbart Fluorierungskatalysatoren, die aus einer Mischung eines Antimontrihalogenids und eines Titantetrahalogenids bestehen. Diese Gemische eignen sich beispielsweise für die Fluorierung von Chlorderivaten des Methans, des Ethans und des Ethylens. Fluorieren kann man jedoch auch entsprechende Brom- oder Jodderivate.

Die Europäische Patentanmeldung EP 0 574 077 A1 offenbart die Herstellung von 1,1,1,2-Tetrafluorethan aus Trifluorethylen durch Reaktion mit HF in flüssiger Phase. Halogenide des Zinns und des Titans werden als mögliche Katalysatoren genannt und in Beispielen verwendet. Die Umsetzung läuft auch ohne Katalysator ab.

A.E. Feiring offenbart in J. Fluorine Chem. 14(1979), Seiten 7 bis 18, daß TiCl₄ die Anlagerung von HF an Tetrachlorethylen katalysiert. Ein anschließender Chlor-Fluor-Austausch führt in geringem Maße zur Bildung von Difluortrichlorethan.

Die britische Patentschrift GB 627 773 A offenbart die Anlagerung von Fluorwasserstoff an bestimmte C2- und C3-Verbindungen. Katalysator ist Zinntetrachlorid und Zinntetrafluorid. Neben der HF-Anlagerung wird auch ein Chlor-Fluor-Austausch beobachtet.

Die WO 95/04021 offenbart die Herstellung von Fluorkohlenwasserstoffen aus Chlorkohlenwasserstoffen, die auch Fluor-substituiert sein können, durch katalysierten Chlor-Fluor-Austausch mit Fluorwasserstoff. Eingesetzt wird vorzugsweise mindestens ein Metallhalogenid ausgewählt aus der Gruppe der Halogenide von Zinn, Titan, Tantal und Antimon. Die Beispiele arbeiten mit Antimonpentafluorid, Titantetrachlorid, Zinntetrachlorid, Antimonpentachlorid bzw. in Abwesenheit eines Katalysators.

Aufgabe der vorliegenden Erfindung ist es, ein Metallhalogenid-Katalysatorsystem mit hoher Aktivität und ein Verfahren zu seiner Anwendung anzugeben. Diese Aufgabe wird durch die vorliegenden Erfindung gelöst.

Der Erfindung liegt die Beobachtung zugrunde, daß Gemische von mindestens teilfluorierten Titanhalogeniden in Kombination mit Zinntetrahalogeniden eine im Vergleich mit den Einzelkomponenten gesteigerte Katalysatoraktivität aufweisen.

Das erfindungsgemäße Verfahren in der Flüssigphase zur Herstellung von organischen Verbindungen mit mindestens einem Fluoratom, wobei mindestens ein Fluoratom durch eine durch Metallhalogenid katalysierte Anlagerung von Fluorwasserstoff an eine ungesättigte C-C-Gruppe oder durch einen durch Metallhalogenid katalysierten Chlor-Fluor-Austausch unter Verwendung von Fluorwasserstoff an mindestens einem vollhalogenierten C-Atom im Ausgangsmaterial eingeführt wird, sieht vor, daß man in Anwesenheit eines mindestens teilweise fluorierten Gemisches von Titanchlorid oder Titanbromid in Kombination mit mindestens teilweise fluoriertem Zinntetrachlorid oder Zinntetrabromid als Metallhalogenid arbeitet, mit der Maßgabe, daß das Atomverhältnis von Titan zu Zinn im Bereich von 9:1 bis 1:9 liegt.

Natürlich kann man das Titan auch in Form eines mindestens teilweise fluorierten Gemisches von Titanchlorid und Titanbromid, das Zinn in Form eines mindestens teilweise fluorierten Gemisches von Zinntetrachlorid und Zinntetrabromid einsetzen. Vorteilhaft geht man aus von Titantetrachlorid und Zinntetrachlorid. Diese Ausführungsform wird im folgenden näher erläutert.

Ganz besonders vorteilhaft ist es, ein Katalysator-Gemisch einzusetzen, welches erhalten wird durch Umsetzen von Titantetrachlorid und Zinntetrachlorid mit Fluorwasserstoff, wobei das Molverhältnis von Fluorwasserstoff zur Summe von Titantetrachlorid und Zinntetrachlorid mindestens 4:1, vorzugsweise mindestens 8:1 beträgt. Besonders gut geeignet sind jene Katalysatorgemische, die erhalten werden, wenn man Titantetrachlorid und Zinntetrachlorid bei einer Temperatur von wenigstens 145 °C während einer Zeitdauer von wenigstens 150 min. mit mindestens der 18-fachen molaren Menge an Fluorwasserstoff bezogen auf die Chlor-Atome der Zinntetrachlorid-Titantetrachlorid-Mischung umsetzt.

Die Katalysatormischung von mindestens teilfluoriertem Zinntetrahalogenid und Titantetrahalogenid kann für jene Herstellungsverfahren eingesetzt werden, in welchen eine Fluorwasserstoff-Anlagerung und/oder ein Chlor-Fluor-Austausch unter Verwendung von Fluorwasserstoff in Anwesenheit eines Titantetrahalogenid-Katalysators oder Zinntetrahalogenid-Katalysators durchgeführt wird. Es gehört deshalb zum Umfang der vorliegenden Erfindung, daß man in bekannten Fluorierungsverfahren, die mittels Zinnhalogenid oder Titanhalogenid katalysiert werden, diesen Katalysator durch die o. a. Gemische von mindestens teilfluoriertem Zinnhalogenid und Titanhalogenid ersetzt.

Besonders gut geeignet ist das erfindungsgemäße Flüssigphasen-Verfahren zur Herstellung von C2-C8-Alkanen, die mindestens ein Fluoratom aufweisen sowie zur Herstellung von C2-C8-Alkanen, die mindestens ein Fluoratom sowie weitere Halogensubstituenten aufweisen. Zu ihrer Herstellung kann man von entsprechenden C2-C8-Alkenen ausgehen und in Anwesenheit der erfindungsgemäßen Katalysatorgemische Fluorwasserstoff addieren.

Das Verfahren ist auch sehr gut geeignet, um C2-C8-Alkane, die ein oder mehrere vollhalogenierte Kohlenstoffatome aufweisen, insbesondere C2-C8-Alkane, die eine oder mehrere CCl₃-, CCl₂F oder CClF₂-Gruppen aufweisen, unter Chlor-Fluor-Austausch unter Verwendung von Fluorwasserstoff in Verbindungen zu überführen, die mindestens ein Fluor-Atom mehr aufweisen als die Ausgangsverbindungen.

Hervorragend geeignet ist das erfindungsgemäße Verfahren zur Herstellung von Alkanen, die mindestens ein Fluor-Atom mehr aufweisen als das eingesetzte Alken, wobei man von Alkenen der Formel CX¹X²=CX³X⁴ ausgeht, worin X¹ für Chlor, Fluor, CCl₃, CCl₂F, CClF₂ oder CF₃ steht und X², X³ und X⁴ unabhängig voneinander Wasserstoff, Chlor oder Fluor bedeuten, und man durch HF-Anlagerung und gegebenenfalls zusätzlichen Chlor-Fluor-Austausch entsprechende Alkane herstellt.

Ganz hervorragend eignet sich das Verfahren zum Chlor-Fluor-Austausch bei Alkanen der Formel CX⁵X⁶X⁷CX⁸X⁹X¹⁰, worin X⁵ für CCl₃, CCl₂F, CClF₂ oder Chlor steht, X⁶ und X⁷ für Chlor oder Fluor stehen, oder, sofern X⁵ nicht Chlor ist, X⁶ und X⁷ unabhängig voneinander für Chlor, Fluor oder Wasserstoff stehen, und X⁸, X⁹ und X¹⁰ unabhängig voneinander für Wasserstoff, Chlor oder Fluor stehen.

Beispielsweise kann man Perchlorethylen zu Fluortetrachlorethan, Difluortrichlorethan bzw. Trifluordichlorethan umsetzen. Man kann beispielsweise auch 1-Fluor-1,1,2,2-tetrachlorethan nach dem erfindungsgemäßen Verfahren zu 1,1-Difluor-1,2,2-trichlorethan und 1,1,1-Trifluor-2,2-dichlorethan umsetzen.

Das Atomverhältnis von Titan zu Zinn liegt vorzugsweise im Bereich von 2:1 bis 1:2. Dabei hat es sich gezeigt, daß ein solches Gemisch mit einem Molverhältnis von Titan zu Zinn im Bereich von 2:1 bis 1:2 nicht nur die HF-Anlagerung an Doppel- oder Dreifachbindungen gut katalysiert; auch der Chlor-Fluoraustausch an CCl₃-, CCl₂F- bzw. CF₂Cl-Gruppen unter Bildung von CF₃-Gruppen wird möglich.

Das Molverhältnis des Edukts zur Summe des Katalysatorgemisches liegt zwischen 1:0,1 und 1:5.

Die Temperatur bei der Umsetzung liegt vorteilhaft im Bereich von 30 bis 250 °C. Der Druck wird so eingeregelt, daß man in der flüssigen Phase arbeitet. Arbeitet man im Autoklaven, baut sich ein autogener Druck, bedingt durch die Anwesenheit von Fluorwasserstoff und gegebenenfalls freigesetztem HCl, auf. Der sich entwickelnde autogene Druck ist natürlich auch abhängig von der Temperatur der Reaktionsmischung. Zweckmäßig kann man den Druck so einregeln, daß er in einem Bereich von 2 bis 80 bar (absolut) liegt.

Ein weiterer Gegenstand der vorliegenden Erfindung ist ein mindestens teilweise fluoriertes Gemisch von Titantetrachlorid und Zinntetrachlorid mit einem Atomverhältnis von Titan zu Zinn im Bereich von 9:1 bis 1:9, insbesondere 2:1 bis 1:2, erhältlich durch Umsetzen von Titantetrachlorid und Zinntetrachlorid mit Fluorwasserstoff, wobei das Molverhältnis von HF zu der Summe von Titantetrachlorid und Zinntetrachlorid mindestens 4:1, vorzugsweise mindestens 8:1 beträgt. Ein solches Gemisch kann als Katalysator für Fluorwasserstoffanlagerungen an C-C-Doppelbindungen und C-C-Dreifachbindungen sowie als Katalysator für den Chlor-Fluor-Austausch unter Verwendung von Fluorwasserstoff als Fluor-Quelle bei Trichlormethyl-, Fluordichlormethyl- und Difluorchlormethyl-Gruppen verwendet werden, insbesondere im Hinblick auf die Herstellung von Verbindungen mit der CF₃-Gruppe.

Vorteil des erfindungsgemäßen Verfahrens ist der hohe Umsatz und die hohe Ausbeute sowie die hohe Selektivität, sowie in bevorzugten Ausführungsformen auch die Möglichkeit, CF₃-Gruppen mit Ti/Sn-Katalysatoren herzustellen.

Die folgenden Beispiele und Vergleichsbeispiele sollen die Erfindung weiter erläutern, ohne sie in ihrem Umfang einzuschränken.

### Beispiele

### Beispiel 1:

### Allgemeine Vorschrift zur Vorfluorierung des Katalysators bzw. der Katalysatormischungen

Die angegebenen Katalysatormengen wurden in einem 250 ml Autoklaven der Firma Roth in einem Aluminiuminliner mit der 20-fachen molaren Menge Fluorwasserstoff versetzt und bei der angegebenen Reaktionstemperatur 3 h vorfluoriert. Die überschüssige HF und die entstandene HCl wurden nach Beendigung der Vorfluorierung und nach dem Abkühlen des Autoklaven in einem Wäscher abgelassen. Beim Einsatz von reinem TiCl₄ als Katalysator erhielt man nach der Vorfluorierung TiCl_{0,1}F_{3,9}, reines SnCl₄ wurde zu SnCl_{2,7}F_{1,3} fluoriert, d.h. die Chloratome sind durch die Vorfluorierungsprozedur nur teilweise durch Fluoratome ausgetauscht worden.

Die Vorfluorierung in einem Autoklaven ohne Aluminiuminliner lieferten vergleichbare Ergebnisse, ebenso die Synthesen von Halogenkohlenwasserstoffen.

### Beispiel 2:

### Hydrofluorierung von Perchlorethylen mit reinem TiCl₄ als Katalysator (Vergleichsbeispiel)

Für die Vorfluorierung wurden 23,4 g (0,123 mol) TiCl₄ eingesetzt. Nach der Vorfluorierung der Katalysatormischung wurden 78,4 g (0,473 mol) Tetrachlorethylen (PCE) und anschließend 100,0 g (4,998 mol) HF in den Autoklaven gegeben und der Autoklav auf eine Innentemperatur von 150 °C im Ölbad erhitzt. Eine nach 3 h Reaktionszeit aus der Gasphase über eine Waschflasche genommene Analysenprobe (Angabe in GC-Flächenprozent) bestand nur aus 21,1 % CF₂Cl-CHCl₂ (122) und 78,9 % Edukt Perchlorethylen (PCE). Die Temperatur wurde über Nacht bei 150 °C gehalten und der Autoklaveninhalt nach dem Abkühlen quantitativ in Eiswasser überführt. Die organische Phase enthielt als Reaktionsprodukte 19,5 % 122 und 80,5 % 121. Der Umsatz an PCE betrug 12,2 %.

### Beispiel 3:

Für die Vorfluorierung wurden 43,2 g( 0,228 mol) TiCl₄ als Mischung mit 6,6 g (0,025 mol) SnCl₄ eingesetzt. Nach der Vorfluorierung der Katalysatormischung wurden 77,9 g (0,470 mol) Tetrachlorethylen (PCE) und anschließend 97,8 g (4,888 mol) HF in den Autoklaven gegeben und der Autoklav auf eine Innentemperatur von 150 °C im Ölbad erhitzt. Eine nach 3 h Reaktionszeit aus der Gasphase über eine Waschflasche genommene Analysenprobe (Angabe in GC-Flächenprozent) bestand zu 0,086 % aus CF₃CHCl₂ (123), 18,894 % CF₂Cl-CHCl₂ (122), 25,382 % CFCl₂-CCl₂H (121) und 55,638 % Edukt Perchlorethylen (PCE). Die Temperatur wurde über Nacht bei 150 °C gehalten und der Autoklaveninhalt nach dem Abkühlen quantitativ in Eiswasser überführt. Die organische Phase enthielt als Reaktionsprodukte 0,6 % 123, 67,4 % 122 und 32,0 % 121. Der Umsatz an PCE betrug 67,1 %.

### Beispiel 4:

### Hydrofluorierung von Perchlorethylen (PCE) mit molarer 1:1-Mischung aus TiCl₄ und SnCl₄

Für die Vorfluorierung wurden 11,5 g (0,061 mol) TiCl₄ als Mischung mit 16,0 g (0,061 mol) SnCl₄ eingesetzt. Nach der Vorfluorierung der Katalysatormischung wurden 50,5 g (0,305 mol) Tetrachlorethylen (PCE) und anschließend 96,2 g (4,808 mol) HF in den Autoklaven gegeben und der Autoklav auf eine Innentemperatur von 150 °C im Ölbad erhitzt. Eine nach 3 h Reaktionszeit aus der Gasphase über eine Waschflasche genommene Analysenprobe (Angabe in GC-Flächenprozent) bestand zu 2,09 % aus CF₃H (23), 0,37 % 22 (CF₂HCl), 7,6 % CF₃CHCl₂ (123), 80,52 % CF₂Cl-CHCl₂ (122), 2,28 % CFCl₂-CCl₂H (121) und 7,14 % Edukt Perchlorethylen (PCE). Die Temperatur wurde über Nacht bei 150 °C gehalten und der Autoklaveninhalt nach dem Abkühlen quantitativ in Eiswasser überführt. Die organische Phase enthielt als Reaktionsprodukte 4,97 % 123, 90,67 % 122 und 4,36 % 121. Der Umsatz an PCE betrug 86,9 %.

### Beispiel 5:

### Hydrofluorierung von Perchlorethylen mit molarer TiCl₄/SnCl₄-Mischung von 1:9 als Katalysator

Für die Vorfluorierung wurden 4,7 g (0,025 mol) TiCl₄ als Mischung mit 58,2 g (0,223 mol) SnCl₄ eingesetzt. Nach der Vorfluorierung der Katalysatormischung wurden 77,9 g (0,470 mol) Tetrachlorethylen (PCE) und anschließend 96,3 g (4,813 mol) HF in den Autoklaven gegeben und der Autoklav auf eine Innentemperatur von 150 °C im Ölbad erhitzt. Eine nach 3 h Reaktionszeit aus der Gasphase über eine-Waschflasche genommene Analysenprobe (Angabe in GC-Flächenprozent) bestand zu 0,031 % aus CF₃CHCl₂ (123), 5,32 % CF₂Cl-CHCl₂ (122), 6,07 % CFCl₂-CCl₂H (121) und 88,58 % Edukt Perchlorethylen (PCE). Die Temperatur wurde über Nacht bei 150 °C gehalten und der Autoklaveninhalt nach dem Abkühlen quantitativ in Eiswasser überführt. Die organische Phase enthielt als Reaktionsprodukte 0,3 % 123, 57,4 % 122 und 42,3 % 121. Der Umsatz an PCE betrug 19,3 %.

### Beispiel 6 (Vergleichsbeispiel):

### Hydrofluorierung von Perchlorethylen mit reinem SnCl₄ als Katalysator

Für die Vorfluorierung wurden 33,5 g (0,129 mol) SnCl₄ eingesetzt. Nach der Vorfluorierung der Katalysatormischung wurden 76,6 g (0,462 mol) Tetrachlorethylen (PCE) und anschließend 99,4 g (4,968 mol) HF in den Autoklaven gegeben und der Autoklav auf eine Innentemperatur von 150 °C im Ölbad erhitzt. Eine nach 3 h Reaktionszeit aus der Gasphase über eine Waschflasche genommene Analysenprobe (Angabe in GC-Flächenprozent) enthielt 0,066 % CF₃CHCl₂ (123), 11,743 % CF₂Cl-CHCl₂ (122), 6,946 % CFCl₂-CCl₂H (121) und 81,16 % Edukt Perchlorethylen (PCE). Die Temperatur wurde über Nacht bei 150 °C gehalten und der Autoklaveninhalt nach dem Abkühlen quantitativ in Eiswasser überführt. Die organische Phase enthielt als Reaktionsprodukte 0,45 % 123, 70,5 % 122 und 29,05 % 121. Der Umsatz an PCE betrug 20,0 %.

### Beispiel 7:

### Herstellung von 1,1,1-Trifluor-2,2-dichlorethan aus 1,1-Difluor-1,2,2-trichlorethan mit molarer 1:1-Mischung aus TiCl₄ und SnCl₄ als Katalysator

Für die Vorfluorierung wurden 22,6 g (0,119 mol) TiCl₄ als Mischung mit 32,0 g (0,123 mol) SnCl₄ eingesetzt. Nach der Vorfluorierung der Katalysatormischung wurden 101 g (0,596 mol) CF₂Cl-CHCl₂ (122) und anschließend 49,9 g (2,495 mol) HF in den Autoklaven gegeben und der Autoklav auf eine Innentemperatur von 140 °C im Ölbad erhitzt. Eine nach 3 h Reaktionszeit aus der Gasphase über eine Waschflasche genommene Analysenprobe (Angabe in GC-Flächenprozent) enthielt 21 % CF₃CHCl₂ (123) und 79 % das Edukt CF₂CI-CHCI₂ (122). Die Temperatur wurde über Nacht bei 140 °C gehalten und der Autoklaveninhalt nach dem Abkühlen quantitativ in Eiswasser überführt. Die organische Phase enthielt als Reaktionsprodukt nur 123. Der Umsatz der Reaktion betrug jedoch nur 7 %.

### Beispiel 8: (Vergleichsbeispiel)

### Versuch zur Herstellung von R123 aus R122 mit SnCl₄ als Katalysator

Durchführung wie Beispiel 7, jedoch mit reinem SnCl₄ als Katalysator. Nach Aufarbeiten der Reaktionsmischung wurde als Produkt nur 123 gefunden. Der Umsatz betrug jedoch nur 0,9 %.

### Beispiel 9: (Vergleichsbeispiel)

### Versuch zur Herstellung von R123 aus R122 mit TiCl₄ als Katalysator

Durchführung wie Beispiel 8, jedoch mit reinem TiCl₄ als Katalysator. Nach Aufarbeiten der Reaktionsmischung wurde kein Produkt gefunden.

## Patentansprüche

1. Flüssigphasenverfahren zur Herstellung von organischen Verbindungen mit mindestens einem Fluoratom, wobei mindestens ein Fluoratom durch eine durch Metallhalogenid katalysierte Anlagerung von HF an eine ungesättigte C-C-Gruppe oder durch einen durch Metallhalogenid katalysierten Chlor-Fluor-Austausch unter Verwendung von HF an mindestens einem vollhalogenierten C-Atom im Ausgangsmaterial eingeführt wird, wobei man in Anwesenheit eines mindestens teilweise fluorierten Gemisches von Titanchlorid oder Titanbromid in Kombination mit mindestens teilweise fluoriertem Zinntetrachlorid oder Zinntetrabromid als Metallhalogenid arbeitet, mit der Maßgabe, daß das Atomverhältnis von Titan zu Zinn im Bereich von 9:1 bis 1:9 liegt.

2. Verfahren nach Anspruch 1, dadurch gekennzeichnet, daß man ein Katalysatorgemisch einsetzt, welches erhalten wird durch Umsetzen von TiCl₄ und SnCl₄ mit HF, wobei das Molverhältnis von HF: (TiCl₄+SnCl₄) mindestens 4:1 beträgt.

3. Verfahren nach Anspruch 1, dadurch gekennzeichnet, daß man C2-C8-Alkane mit mindestens einem Fluoratom oder C2-C8-Alkane mit mindestens einem Fluoratom und weiteren Halogensubstituenten herstellt, indem man an entsprechende C2-C8-Alkene HF addiert.

4. Verfahren nach Anspruch 1, dadurch gekennzeichnet, daß man C2-C8-Alkane mit einer oder mehreren CCl₃-, CCI₂F- oder CClF₂-Gruppen einsetzt und unter Chlor-Fluor-Austausch unter Verwendung von HF in Verbindungen überführt, die mindestens 1 Fluor-Atom mehr aufweisen als die Ausgangsverbindungen.

5. Verfahren nach Anspruch 3, dadurch gekennzeichnet, daß man Alkene der Formel CX¹X²=CX³X⁴ einsetzt, worin X¹ für Chlor, Fluor, CCl₃, CCl₂F, CClF₂ oder CF₃ steht und X², X³ und X⁴ unabhängig voneinander Wasserstoff, Chlor oder Fluor bedeuten, und man durch HF-Anlagerung und gegebenenfalls zusätzlichen Chlor-Fluor-Austausch Alkane herstellt, die mindestens 1 Fluoratom mehr als das eingesetzte Alken aufweisen.

6. Verfahren nach Anspruch 4, dadurch gekennzeichnet, daß man Alkane der Formel CX⁵X⁶X⁷CX⁸X⁹X¹⁰ einsetzt, worin X⁵ für CCl₃, CCl₂F, CClF₂ oder Chlor steht, X⁶ und X⁷ für Chlor oder Fluor stehen, und sofern X⁵ nicht Chlor ist, auch jeweils Wasserstoff bedeuten können, und X⁸, X⁹ und X¹⁰ unabhängig voneinander für Wasserstoff, Chlor oder Fluor stehen.

7. Verfahren nach Anspruch 5 oder 6, dadurch gekennzeichnet, daß man Perchlorethylen, 1-Fluor-1,1,2,2-Tetrachlorethan oder 1,1-Difluor-1,2,2-trichlorethan einsetzt.

8. Verfahren nach einem der vorhergehenden Ansprüche, dadurch gekennzeichnet, daß das Atomverhältnis von Titan zu Zinn im Bereich von 9:1 bis 1:9 liegt.

9. Verfahren nach Anspruch 1, dadurch gekennzeichnet, daß man bei einem Druck von 2 bis 80 bar (absolut) und einer Temperatur von 30 bis 250 °C arbeitet, wobei man den Druck so einregelt, daß man in der Flüssigphase arbeitet.

10. Verfahren nach Anspruch 1, dadurch gekennzeichnet, daß zur Herstellung von Verbindungen mit einer CF₃-Gruppe durch Chlor-Fluor-Austausch an einer CCl₃-, CFCl₂- oder CF₂Cl-Gruppe das Atomverhältnis von Titan zu Zinn im Bereich von 1:2 bis 2:1 liegt.

11. Mindestens teilweise fluoriertes Gemisch von Titantetrachlorid und Zinntetrachlorid mit einem Atomverhältnis von Titan zu Zinn im Bereich von 1:9 bis 9:1, erhältlich durch Umsetzen von Titantetrachlorid und Zinntetrachlorid mit Fluorwasserstoff, wobei das Molverhältnis von HF zu der Summe von Titantetrachlorid und Zinntetrachlorid mindestens 4:1 beträgt.

12. Verwendung des mindestens teilfluorierten Gemisches gemäß Anspruch 11 als Katalysator für HF-Anlagerungen an C-C-Doppelbindungen und C-C-Dreifachbindungen, und als Katalysator für den Chlor-Fluor-Austausch unter Verwendung von HF bei CCl₃-, CCl₂F-, und CCIF₂-Gruppen.

## Claims

1. A liquid-phase process for the production of organic compounds having at least one fluorine atom, in which at least one fluorine atom is introduced by addition, catalysed by metal halide, of HF to an unsaturated C-C group or by a chlorine-fluorine exchange, catalysed by metal halide, using HF on at least one fully halogenated C atom in the starting material, wherein one operates in the presence of an at least partially fluorinated mixture of titanium chloride or titanium bromide in combination with at least partially fluorinated tin tetrachloride or tin tetrabromide as metal halide, with the proviso that the atomic ratio of titanium to tin is in the range of 9:1 to 1:9.

2. A process according to Claim 1, characterised in that a catalyst mixture is used which is obtained by reacting TiCl₄ and SnCl₄ with HF, the molar ratio of HF:(TiCl₄+SnCl₄) being at least 4:1.

3. A process according to Claim 1, characterised in that C2-C8 alkanes having at least one fluorine atom or C2-C8 alkanes having at least one fluorine atom and additional halogen substituents are produced by adding HF to appropriate C2-C8 alkenes.

4. A process according to Claim 1, characterised in that C2-C8 alkanes having one or more CCl₃, CCl₂F or CClF₂ groups are used, and with chlorine-fluorine exchange using HF are converted into compounds which have at least one fluorine atom more than the starting compounds.

5. A process according to Claim 3, characterised in that alkenes of the formula CX¹X²=CX³X⁴ are used, wherein X¹ stands for chlorine, fluorine, CCl₃, CCl₂F, CClF₂ or CF₃, and X², X³ and X⁴ independently of each other stand for hydrogen, chlorine or fluorine, and by HF addition and optionally additional chlorine-fluorine exchange alkanes are produced which have at least one fluorine atom more than the alkene used.

6. A process according to Claim 4, characterised in that alkanes of the formula CX⁵X⁶X⁷CX⁸X⁹X¹⁰ are used, wherein X₅ stands for CCl₃, CCl₂F, CClF₂ or chlorine, X⁶ and X⁷ stand for chlorine or fluorine, and if X⁵ is not chlorine, may also each stand for hydrogen, and X⁸, X⁹ and X¹⁰ independently of each other stand for hydrogen, chlorine or fluorine.

7. A process according to Claim 5 or 6, characterised in that perchloroethylene, 1-fluoro-1,1,2,2-tetrachloroethane or 1,1-difluoro-1,2,2-trichloroethane is used.

8. A process according to one of the preceding Claims, characterised in that the atomic ratio of titanium to tin is in the range of 9:1 to 1:9.

9. A process according to Claim 1, characterised in that one operates at a pressure of 2 to 80 bar (absolute) and a temperature of 30 to 250°C, the pressure being adjusted such that one operates in the liquid phase.

10. A process according to Claim 1, characterised in that for the production of compounds having a CF₃ group by chlorine-fluorine exchange on a CCl₃, CFCl₂ or CF₂Cl group the atomic ratio of titanium to tin is in the range of 1:2 to 2:1.

11. An at least partially fluorinated mixture of titanium tetrachloride and tin tetrachloride having an atomic ratio of titanium to tin in the range of 1:9 to 9:1, obtainable by reacting titanium tetrachloride and tin tetrachloride with hydrogen fluoride, the molar ratio of HF to the total of titanium tetrachloride and tin tetrachloride being at least 4:1.

12. The use of the at least partially fluorinated mixture according to Claim 11 as a catalyst for HF addition to C-C double bonds and C-C triple bonds, and as a catalyst for the chlorine-fluorine exchange using HF in CCl₃, CCl₂F and CClF₂ groups.

## Revendications

1. Procédé en phase liquide de préparation de composés organiques comprenant au moins un atome de fluor, dans lequel au moins un atome de fluor est introduit par une addition de fluorure d'hydrogène (HF) catalysée par un halogénure de métal à un groupe C-C insaturé ou par un échange chlore - fluor catalysé par un halogénure de métal en utilisant le HF au niveau d'au moins un atome C entièrement halogéné dans un matériau de départ, où l'on travaille en présence d'un mélange au moins partiellement fluoré de chlorure de titane ou de bromure de titane en combinaison avec au moins un tétrachlorure d'étain ou un tétrabromure d'étain partiellement fluoré en tant qu'halogénure de métal, avec comme prérequis que le rapport atomique entre titane et étain soit compris dans la gamme allant de 9:1 à 1:9.

2. Procédé selon la revendication 1, caractérisé en ce que l'on utilise un mélange de catalyseurs obtenu par réaction de TiCl₄ et de SnCl₄ avec HF, dans lequel le rapport molaire HF:(TiCl₄ + SnCl₄) est au moins égal à 4:1.

3. Procédé selon la revendication 1, caractérisé en ce que l'on prépare des alcanes en C₂ à C₈ avec au moins un atome de fluor ou des alcanes en C₂ à C₈ avec au moins un atome de fluor et d'autres substituants d'halogène, en ce que l'on ajoute le HF aux alcènes en C₂ à C₈ correspondants.

4. Procédé selon la revendication 1, caractérisé en ce que l'on utilise des alcanes en C₂ à C₈ avec un ou plusieurs groupe(s) CCI₃-, CCI₂F- ou CCIF₂ et que l'on réalise la conversion par échange de chlore - fluor en utilisant du fluorure d'hydrogène en composés qui possèdent au moins un atome de fluor de plus que les composés de départ.

5. Procédé selon la revendication 3, caractérisé en ce que l'on utilise des alcènes de formule CX¹X²=CX³X⁴, où X¹ représente un chlore, un fluor, CCl₃, CCl₂F, CCIF₂ ou CF₃ et X², X³ et X⁴ sont indépendamment l'un de l'autre un atome d'hydrogène, de chlore ou de fluor, et que l'on prépare par addition de HF et éventuellement échange supplémentaire de chlore - fluor des alcanes qui possèdent au moins un atome de fluor de plus que l'alcène utilisé.

6. Procédé selon la revendication 4, caractérisé en ce que l'on utilise des alcanes de formule CX⁵X⁶X⁷CX⁸X⁹X¹⁰, où X⁵ représente CCl₃, CCl₂F, CCIF₂ ou un chlore, X⁶ et X⁷ représentent un chlore ou un fluor, et, si X⁵ n'est pas un chlore, peuvent également représenter respectivement un hydrogène, et X⁸, X⁹ et X¹⁰ sont indépendamment l'un de l'autre un atome d'hydrogène, de chlore ou de fluor.

7. Procédé selon la revendication 5 ou 6, caractérisé en ce que l'on utilise du perchloroéthylène, du 1-fluor-1,1,2,2-tétrachloroéthane ou du 1,1-difluoro-1,2,2-trichloroéthane.

8. Procédé selon l'une quelconque des revendications précédentes, caractérisé en ce que le rapport atomique entre titane et étain est compris dans la gamme allant de 9:1 à 1:9.

9. Procédé selon la revendication 1, caractérisé en ce que l'on travaille à une pression comprise entre 2 et 80 bars (absolue) et à une température comprise entre 30 et 250 °C, la pression étant ajustée de façon telle que l'on travaille en phase liquide.

10. Procédé selon la revendication 1, caractérisé en ce que, pour préparer des composés avec un groupe CF₃- par échange chlore - fluor sur un groupe CCl₃-, CFCl₂- ou CF₂CI-, le rapport atomique entre titane et étain est compris dans la gamme allant de 1:2 à 2:1.

11. Mélange au moins partiellement fluoré de tétrachlorure de titane et de tétrachlorure d'étain selon un rapport atomique entre titane et étain compris dans la gamme allant de 1:9 à 9:1, pouvant être obtenu par réaction de tétrachlorure de titane et de tétrachlorure d'étain avec du fluorure d'hydrogène, dans lequel le rapport molaire entre HF et la somme du tétrachlorure de titane et du tétrachlorure d'étain est au moins égal à 4:1.

12. Utilisation du mélange au moins partiellement fluoré selon la revendication 11 en tant que catalyseur pour les additions de HF aux doubles liaisons C-C et les triples liaisons C-C, et en tant que catalyseur pour l'échange chlore - fluor en utilisant HF au niveau des groupes CCl₃-, CCl₂F- et CClF₂.
